Europäisches Patentamt

European Patent Office

Office européen des brevets

(19)

(11) Veröffentlichungsnummer: **0 201 796 B1**

# EUROPÄISCHE PATENTSCHRIFT

(12)

(45) Veröffentlichungstag der Patentschrift: **25.03.92**

(21) Anmeldenummer: **86105860.0**

(22) Anmeldetag: **28.04.86**

(51) Int. Cl.5: **C12N 1/20**, C12P 1/04,
//A61K39/02,(C12P1/04,
C12R1:01)

(54) Treponema hyodysenteriae-Bacterin und Verfahren für dieses.

(30) Priorität: **10.05.85 US 732872**

(43) Veröffentlichungstag der Anmeldung:
**20.11.86 Patentblatt 86/47**

(45) Bekanntmachung des Hinweises auf die
Patenterteilung:
**25.03.92 Patentblatt 92/13**

(84) Benannte Vertragsstaaten:
**AT BE CH DE FR GB IT LI NL SE**

(56) Entgegenhaltungen:
**EP-A- 155 790**
**US-A- 4 100 272**

**BIOLOGICAL ABSTRACTS, Band 69, Seite
8574, Ref.Nr. 80868, Philadelphia, Pa., US;
R.M. LEMCKE et al.: "Sterol requirement for
the growth of Treponema hyodysenteriae", &
J. GEN MICROBIOL 116(2): 539-544**

**BIOLOGICAL ABSTRACTS/RRM, Band 29,
1985, Ref.Nr. 32741, Philadelphia, Pa., US;
"Metabolism of blood cells and cholesterol
by Treponema-hyodysenteriae"**

(73) Patentinhaber: **MOBAY CORPORATION
Patent Department Mobay Road
Pittsburgh Pennsylvania 15205-9741(US)**

(72) Erfinder: **Parizek, Richard E.
8202 Noland Road
Lenexa Kansas(US)**

(74) Vertreter: **Schumacher, Günter, Dr. et al
c/o Bayer AG Konzernverwaltung RP Patentabteilung
W-5090 Leverkusen 1 Bayerwerk(DE)**

**Beschreibung**

Schweine-Dysenterie ist eine schwere mukohämorrhagische Diarrhöe, die in erster Linie Schweine nach dem Absetzen befällt. Die Bekämpfung dieser Krankheit erfolgt im allgemeinen durch den Einsatz von Chemotherapeutika und Antibiotika. Bisher gibt es noch kein wirksames nichtmedikamentöses vorbeugendes Mittel zur Bekämpfung dieser Krankheit. Die anaerobe Spirochäte Treponema hyodysenteriae wird als das primäre ätiologische Agens der Schweine-Dysenterie angesehen. Die US-PS 4 100 272 offenbart die Verwendung eines abgetötete Zellen von T. hyodysenteriae enthaltenden Impfstoffs zur Erhöhung der Widerstandsfähigkeit von Schweinen gegen die Schweine-Dysenterie.

Dieses Verfahren hat bis heute keine technisch-wirtschaftliche Akzeptanz gefunden, da das Impfstoff-Produkt nur relativ niedrige Aktivität besitzt. Die beschriebene Behandlungsvorschrift umfaßt sechs intravenöse Injektionen. Es ist erwünscht, einen Impfstoff zu haben, der gemäß einer Behandlungsvorschrift angewandt werden kann, die eine geringere Zahl intramuskulärer Injektionen vorsieht.

Es ist bekannt, daß an Cholesterin reiche Fraktionen als Wachstumsförderer für einige Organismen einsetzbar sind. J. Bacteriol. 135 (3), 818-827 (1978), beschreibt die Verwendung einer cholesterinreichen Fraktion als Wachstumsförderer für Mycoplasma pneumoniae und Mycoplasma arthritidis. J. Gen. Microbiology 116, 539-543 (1980), beschreibt die Verwendung von USP-Cholesterin beim Wachstum von T. hyodysenteriae.

Es gibt keinen bekannten Stand der Technik, der die spezielle Verwendung cholesterinreicher boviner Fraktionen zur Erhöhung der Ausbeute an Zellen von T. hyodysenteriae für einen nachfolgenden Einschluß in einen Impfstoff nahelegt, der zur Bekämpfung der Schweine-Dysenterie geeignet ist.

Gemäß der vorliegenden Erfindung wird ein Verfahren zur Kultivierung von Treponema hyodysenteriae in einem geeigneten Nährmedium für eine nachfolgende Verwendung der gewonnenen Zellen in einem Impfstoff verfügbar gemacht, wobei das Nährmedium eine cholesterinreiche bovine Fraktion enthält.

Das Verfahren der vorliegenden Erfindung kann mit jedem virulenten Stamm von T. hyodysenteriae durchgeführt werden. Ein virulenter Stamm ist ein solcher, der zur Erzeugung einer typischen Schweine-Dysenterie-Infektion befähigt ist. Zwei besondere Stämme (B204 und B234) haben sich für diesen Zweck als geeignet erwiesen. Diese wurden bei der American Type Culture Collection hinterlegt und haben die Bezeichnungen ATCC Nr. 31212 (B204) und ATCC Nr. 31287 (B234).

Der T. hyodysenteriae-Stamm kann in Medien kultiviert werden, die eine Mischung aus tryptischer Sojabrühe (Difco Laboratories) ergänzt mit fötalem Kälberserum oder Lammserum, Dextrose, L-Cystein-HCl, Vitamin B 12 und Hefeextrakt umfassen. Die Verbesserung der vorliegenden Erfindung besteht darin, eine cholesterinreiche bovine Fraktion in solche Medien für das Wachstum von T. hyodysenteriae-Zellen einzubeziehen. Die cholesterinreiche bovine Fraktion ist vorzugsweise in einer Menge von 1,0 bis 2,5 Vol.-%, bezogen auf das Volumen des Mediums, anwesend, jedoch kann sie in einer Menge von 0,1 bis 5,0 Vol.-% verwendet werden.

Cholesterinreiche bovine Fraktionen, die für den Einsatz bei dem Verfahren der vorliegenden Erfindung geeignet sind, sind von verschiedenen Quellen erhältlich. Cholesterol Concentrate Code 82-010, das bei der Miles Scientific Division der Miles Laboratories, Inc., erhältlich und aus Rinderserum entsprechend dem in der US-PS 4 290 774 offenbarten und beanspruchten Verfahren hergestellt ist, ist brauchbar. Bovine Cholesterol Concentrate List 3200, das bei Biocell Laboratories erhältlich ist, ist ein anderes geeignetes Material. Die bevorzugte cholesterinreiche bovine Fraktion, die in dem Verfahren der vorliegenden Erfindung verwendbar ist, wird mittels eines Verfahrens hergestellt, das die folgenden Schritte umfaßt:

(a) In-Berührung-Bringen eines flüssigen, Cholesterin enthaltenden bovinen Plasmas oder Serums oder einer Fraktion daraus mit einem Siliciumdioxid-Adsorptionsmittel, um das Cholesterin zu adsorbieren;

(b) Abtrennen des adsorbierten Cholesterins aus dem verbleibenden flüssigen Plasma oder Serum;

(c) Einfrieren und Auftauen des adsorbierten Cholesterins;

(d) Eluieren des adsorbierten Cholesterins bei einem pH von 9,0 bis 11,5;

(e) Konzentrieren der eluierten Cholesterin-Lösung durch Ultrafiltration;

(f) Einstellen des pH der konzentrierten Cholesterin-Lösung auf einen Wert im Bereich von 11,0 bis 11,4;

(g) Dialysieren der konzentrierten Cholesterin-Lösung nacheinander gegen Natriumcarbonat und Wasser;

(h) Weiteres Konzentrieren der dialysierten Cholesterin-Lösung durch Ultrafiltration auf einen Cholesterinspiegel von 50 bis 3000 mg/dl;

(i) Einstellen des pH der konzentrierten Cholesterin-Lösung auf einen Wert im Bereich von 7,0 bis 11,0;

(j) Erhitzen der konzentrierten Cholesterin-Lösung auf 50°C bis 100°C für die Dauer von 30 min bis 24 h; und

(k) Isolieren einer gereinigten cholesterinreichen bovinen Fraktion daraus.

Diese spezielle Fraktion und das Verfahren zu ihrer Gewinnung werden in der gleichzeitig anhängigen

EP 0 201 796 B1

US-Patentanmeldung Serial No. 732856 beschrieben und beansprucht, die gleichzeitig hiermit eingereicht und auf denselben Anmelder übertragen wurde.

Das Ausgangsmaterial für den Einsatz bei der Gewinnung der cholesterinreichen Fraktion kann ein beliebiges Rinderblut-Plasma oder -Serum oder eine Fraktion desselben sein, das bzw. die Cholesterin enthält. Das bevorzugte Ausgangsmaterial ist Rinderserum. Wenn das Ausgangsmaterial Serum ist, wird bevorzugt, ein lösliches Salz wie Natriumcitrat bis zu einer Ionenstärke von 0,25 bis 1,0 zuzusetzen. Zu anderen geeigneten Salzen zählen Natriumchlorid, Natriumphosphat, Kaliumphosphat, Ammoniumsulfat und Natriumsulfat. Der Zusatz eines löslichen Salzes zu der oben genannten Konzentration erhöht die Menge des in dem nachfolgenden Schritt der Adsorption an Siliciumdioxid adsorbierten Cholesterins. Rinderplasma wird im allgemeinen nach einer Methode gesammelt, die den Zusatz von Citrat als Anticoagulans einschließt. Diese Salz-Konzentration ist gewöhnlich für den Adsorptionsschritt ausreichend, und zusätzliches Salz wird nicht mehr benötigt.

Das Plasma- oder Serum-Ausgangsmaterial wird bei einer Temperatur von 0°C bis 50°C, vorzugsweise bei 20°C bis 25°C, gehalten. Der pH wird auf einen Bereich von 5,5 bis 9,0, vorzugsweise von 7,0 bis 8,0, eingestellt.

Das bei diesem Verfahren einsetzbare Siliciumdioxid-Adsorptionsmittel hat keinerlei kritische Zusammensetzung. Geeignete Siliciumdioxid-Materialien sind das bei der Cabot-Corporation unter der Handelsbezeichnung Cabosil® erhältliche mikrofeine Siliciumdioxid und das bei der Cary Company unter der Handelsbezeichnung Aerosil 380® erhältliche pulverisierte Siliciumdioxid. Das Siliciumdioxid wird dem flüssigen Plasma oder Serum in einer Menge von 2 bis 50 g/l, vorzugsweise von 10 bis 20 g/l, zugesetzt.

Die Siliciumdioxid-Suspension in dem flüssigen Plasma oder Serum wird dann etwa 3 bis 4 h vermischt. Das das adsorbierte Cholesterin enthaltende Siliciumdioxid wird dann von dem verbleibenden flüssigen Plasma oder Serum abgetrennt, vorzugsweise durch Zentrifugieren, und die flüssige Phase wird verworfen. Die Siliciumdioxid-Paste wird bei -20°C eingefroren und wenigstens ein Woche, vorzugsweise zwei Wochen, bei dieser Temperatur gehalten. Die gefrorene Paste wird dann 24 bis 48 h bei Raumtemperatur (etwa 20°C bis 25°C) aufgetaut. Etwa aus der aufgetauten Paste ausgedrückte Flüssigkeit wird verworfen.

Die Siliciumdioxid-Paste wird zur Entfernung unerwünschter Proteine gewaschen. Dies geschieht durch Suspendieren der Paste in einer etwa 0,15 M Natriumchlorid enthaltenden wäßrigen Salz-Lösung. Andere geeignete Salze sind Natriumacetat und Natriumphosphat. Die Salzlösung wird in einer Menge von etwa zwei Mal dem Gewicht der Paste verwendet. Die Paste wird von der Flüssigkeit getrennt. Dieser Vorgang des Waschens mit einer Salz-Lösung wird vorzugsweise zur Entfernung okkludierter Proteine zweimal wiederholt.

Die gewaschene Paste wird in etwa 2 Volumina entionisiertem oder destilliertem Wasser suspendiert, und der pH wird durch Zugabe geeigneten alkalischen Materials wie wäßrigem Natriumhydroxid auf 9,0 bis 11,5, vorzugsweise auf 10,4 bis 10,6, eingestellt. Die Suspension wird etwa 2 h gerührt, wobei während dieser Zeit der pH durch periodische Zugabe des obigen alkalischen Materials auf dem gewünschten Wert gehalten wird. Diese Behandlung eluiert die gewünschte Cholesterin-Fraktion von dem Siliciumdioxid. Danach läßt man die Suspension 12 bis 24 h, vorzugsweise 12 bis 18 h, absitzen. Der das Cholesterin enthaltende Überstand wird abgehebert für die Weiterbehandlung. Es wird bevorzugt, nur dieses erste Eluat für die Gewinnung der gewünschten cholesterinreichen Produkt-Fraktion zu verwenden. Es ist jedoch möglich, die oben genannten Schritte des alkalischen Suspendierens, Eluierens, Rührens und Absitzen weitere zweimal zu wiederholen und die Überstände aus der zweiten und der dritten Elution mit dem Material der ersten Elution zu vereinigen. Das Siliciumdioxid wird verworfen.

Die Cholesterin-Lösung wird durch Filtrieren und Zentrifugieren zur Entfernung etwaiger Spuren Siliciumdioxid geklärt und dann mittels Techniken der Ultrafiltration auf 15 bis 50 %, vorzugsweise auf 20 %, ihres Anfangsvolumens konzentriert. Der pH wird durch Alkali-Zusatz auf einen Wert in dem Bereich von 11,0 bis 11,4, vorzugsweise auf pH 11,2, eingestellt. Dies trägt zur Solubilisierung etwaigen restlichen Siliciumdioxids zum Zwecke der anschließenden Entfernung desselben bei.

Das Cholesterin-Konzentrat wird dann einer Behandlung zur Entfernung von im wesentlichen der Gesamtmenge der vorhandenen Salze unterworfen. Die bevorzugte Arbeitsweise zur Entfernung der Salze besteht darin, nacheinander gegen Natriumcarbonat und gegen Wasser zu dialysieren. Dieser Schritt der Entfernung der Salze ist wichtig wegen des anschließenden Schrittes der Wärmebehandlung. Eine signifikante Menge Salz bewirkt eine unerwünschte Denaturierung des Cholesterins beim Erhitzen.

Die dialysierte Cholesterin-Lösung wird durch Ultrafiltration weiter konzentriert auf einen Cholesterin-Wert von 50 bis 3000 mg/dl, vorzugsweise 1000 bis 2000 mg/dl.

Der pH der konzentrierten Cholesterin-Lösung wird dann auf einen Wert in dem Bereich von 7,0 bis 11,0, vorzugsweise auf pH 7,6, eingestellt, um sie für den nachfolgenden Einsatz in einem Wachstumsmedium besonders verträglich zu machen.

Die Lösung wird 30 min bis 24 h, vorzugsweise 30 bis 60 min, auf 50°C bis 100°C, vorzugsweise auf 80°C, erhitzt, um die Lagerbeständigkeit des Cholesterins zu erhöhen. Die Lösung wird auf Raumtemperatur abgekühlt und steril filtriert, wodurch eine gereinigte cholesterinreiche Fraktion gewonnen wird. Dieses Produkt ist kein reines Cholesterin, sondern es ist mit einer kleineren Menge nicht identifizierter Stoffe vermischt, die das Gewinnungsverfahren mit durchlaufen haben.

Reines Cholesterin ist für die Verwendung in der vorliegenden Erfindung nicht geeignet, da es das Wachstum von T. hyodysenteriae nicht nennenswert in der Weise beeinflußt, wie das bei den cholesterinreichen bovinen Fraktionen der Fall ist.

Die T. hyodysenteriae-Zellen werden in den die cholesterinreiche bovine Fraktion enthaltenden Medien unter Bedingungen kultiviert, die Fachleuten wohlbekannt sind. Die gewonnenen Zellen werden dann in wohlbekannter Weise abgetötet, wobei Mittel wie Formalin, Merthiolate oder beta-Propiolacton verwendet werden. Die Zellen werden dann geerntet und zur Erzeugung eines gewünschten Impfstoffes in einem geeigneten Trägermedium suspendiert.

Die Aktivität des Impfstoffes kann durch den Einsatz eines Adjuvans weiter verstärkt werden. Ein Adjuvans, das in bezug auf den Einsatz mit den T. hyodysenteriae-Zellen bevorzugt ist, die mittels des Verfahrens der vorliegenden Erfindung gewonnen wurden, ist die Kombination eines Acrylsäure-Polymers, das mit einem Polyallylsaccharid vernetzt ist, und eines speziellen Emulsionssystems, das in der US-PS 3 919 411 offenbart und beansprucht ist. Ein solches Adjuvans wird von der Bayvet Division der Miles Laboratories, Inc., unter der Handelsbezeichnung HAVLOGEN® vertrieben.

Es wurde gefunden, daß der in der oben beschriebenen Weise erzeugte T. hyodysenteriae-Impfstoff bei intramuskulärer Injektion von nur zwei Dosen im Abstand von drei Wochen in signifikanter Weise die Widerstandsfähigkeit von Schweinen gegen die Schweine-Dysenterie zu erhöhen vermag.

Die Erfindung wird anhand der folgenden Beispiele näher erläutert.

Beispiel 1

Eine cholesterinreiche bovine Fraktion wurde in folgender Weise hergestellt. Frisches Rinderserum wurde auf eine Temperatur von 20°C bis 25°C gebracht, und 14,7 g/l Natriumcitrat (Serum-Ionenstärke 0,5) wurden hinzugefügt. Die erhaltene Lösung wurde 30 min gerührt, und der pH wurde durch Zusatz einer passenden Menge 1N Natriumhydroxid auf 7,0 eingestellt. Feinteiliges Siliciumdioxid wurde in einer Menge von 10 g/l zugegeben, und die entstandene Aufschlämmung wurde 3 h bei Raumtemperatur gerührt. Das adsorbiertes Material enthaltende Siliciumdioxid wurde dann durch Zentrifugieren von der flüssigen Phase abgetrennt, und die Flüssigkeit wurde verworfen. Die Siliciumdioxid-Paste wurde dann bei -20°C eingefroren und zwei Wochen bei dieser Temperatur aufbewahrt. Die gefrorene Paste wurde dann 48 h bei Raumtemperatur aufgetaut. Die ausgedrückte Flüssigkeit wurde verworfen. Die Siliciumdioxid-Paste wurde dann in 2 Volumina einer 0,85-prozentigen (bezogen auf Gewicht/Volumen) wäßrigen Natriumchlorid-Lösung (0,146M NaCl) suspendiert. Sie wurde 15 min sanft vermischt und wenigstens 3 h absitzen gelassen. Die überstehende Flüssigkeit wurde abgehebert und verworfen. Dieser Schritt des Waschens wurde zweimal wiederholt. Die gewaschene Paste wurde dann in 2 Volumina entionisiertem Wasser suspendiert. Der pH wurde durch Zugabe von 1N Natriumhydroxid auf 10,5 eingestellt. Die erhaltene Suspension wurde 2 h bei Raumtemperatur gerührt, wobei der pH jeweils auf 10,5 nachjustiert wurde. Dann wurde das Rühren beendet, und die Suspension wurde 18 h absitzen gelassen. Der Überstand wurde mittels eines Hebers abgenommen und durch Filtration und Zentrifugieren geklärt. Das Siliciumdioxid wurde verworfen. Die geklärte Lösung wurde dann durch Ultrafiltration auf 20 % ihres Anfangsvolumens konzentriert. Der pH des konzentrierten Materials wurde durch Zusatz von 1N Natriumhydroxid auf pH 11,2 eingestellt. Das konzentrierte Material wurde dann gegen 6 Volumina 0,01M Natriumcarbonat bei pH 11,2 dialysiert. Danach wurde es gegen 6 Volumina entionisiertes Wasser dialysiert. Der Cholesterin-Gehalt des Konzentrats wurde mittels bekannter Methoden analysiert, und das Konzentrat wurde durch Ultrafiltration auf einen Cholesterin-Wert von 1000 mg/dl weiter konzentriert. Der pH wurde dann durch Zugabe von 1N Salzsäure auf 7,6 eingestellt, und die erhaltene Lösung wurde dann 1 h auf 80°C erhitzt. Die Lösung wurde dann auf Raumtemperatur abgekühlt und steril filtriert. Das filtrierte Material wurde dann als eine gereinigte cholesterinreiche bovine Fraktion gewonnen.

Gefrorenes Keimmaterial von T. hyodysenteriae, Stamm ATCC Nr. 31212 wurde in ein Glasröhrchen inokuliert, das 13 ml tryptischer Sojabrühe (Difco Laboratories, 27,5 g/l), 10 % fötales Kälberserum, 0,5 % Dextrose, 0,05 % L-Cystein-HCl und 0,25 $\mu$g/ml Vitamin B 12 enthielt. Die Prozentzahlen beziehen sich auf Gewicht/Volumen. Das Röhrchen wurde dann verschlossen und unter einer anaeroben Atmosphäre, die 10 Vol.-% Wasserstoff, 80 Vol.-% Stickstoff und 10 Vol.-% Kohlenstoffdioxid enthielt, 72 h bei 37°C inkubiert.

Die erhaltene Impfkultur wurde dann in zwei das gleiche Medium enthaltende Röhrchen passagiert, die 24 h bei 37°C unter den gleichen Bedingungen inkubiert wurden. Der Inhalt der Röhrchen wurde vereinigt. Ein Teil von 7 ml der vereinigten aktiven Impfkultur wurde dann in jede von zwei 500 ml-Flaschen gegeben, die jeweils 200 ml tryptischer Sojabrühe, 1 % Hefeextrakt, 5 % Lammserum, 0,5 % Dextrose, 0,05 % L-Cystein-HCl und 0,25 µg/ml Vitamin B 12 enthielten. Die Prozentzahlen beziehen sich auf Gewicht/Volumen. Der Inhalt der Flaschen wurde dann unter der oben beschriebenen anaeroben Atmosphäre bei 37°C bis zum Trübwerden (etwa 24 h) inkubiert.

Die erhaltenen erweiterten Impfkulturen wurden dann in zwei Teile geteilt, und jeder Teil wurde für sich allein zur Inokulation eines von zwei 14 l-Pilot-Fermentern verwendet, die jeweils 7 l tryptischer Sojabrühe, 1 % Hefeextrakt, 3 % Lammserum, 1 % Dextrose, 0,05 % L-Cystein-HCl und 0,25 µg/ml Vitamin B 12 enthielten. Die Prozentzahlen beziehen sich auf Gewicht/Volumen. Der eine der Fermenter wurde weiter ergänzend mit 2,5 Vol.-% der wie oben beschrieben hergestellten cholesterinreichen bovinen Fraktion beschickt. Beide Fermenter wurden dann 44 h bei 37°C unter der obigen anaeroben Atmosphäre inkubiert. Der pH wurde durch periodische Zugaben von 5N Natriumhydroxid so gesteuert, daß er bei 6,5 lag.

Bei Beendigung der vorstehenden Inkubation wurden aus jedem Fermenter geeignete Proben entnommen, und die Fermenter-Inhalte wurden dann mit 0,15 Vol.-% Formalin behandelt, um die Zellen zu inaktivieren. Die Zellproben wurden mikroskopisch untersucht, die Gesamtzahl der Zellen wurde in einer Petroff-Hauser-Zählkammer ermittelt, und die Zahl der lebensfähigen Zellen wurde durch serielle Verdünnung auf frisch gegossenen 5 % Rinderblut-Agar-Platten bestimmt. Die Blut-Agar-Platten wurden 4 d bei 37°C unter einer anaeroben Atmosphäre aus 50 Vol.-% Wasserstoff und 50 Vol.-% Kohlenstoffdioxid inkubiert. Die T. hyodysenteriae-Kolonien wurden als individuelle Zonen der Hämolyse beobachtet. Die Ergebnisse dieser Untersuchungen sind in der folgenden Tabelle 1 zusammengestellt.

und ohne cholesterinreiche bovine Fraktion

| Medium | Mikroskopische Untersuchung | Gesamt- Zahl der Zellen | Zahl der lebensfähigen Zellen |
|---|---|---|---|
| ohne Cholesterin-Fraktion | locker gewundene, relativ lange, dünnwandige Zellen, allgemein inaktiv. | $1,3 \times 10^9$ pro ml | $3,7 \times 10^7$ pro ml |
| mit Cholesterin-Fraktion | dicht gewundene, kürzere Zellen mit dickerer Wand, aktiv. | $2,6 \times 10^9$ | $1,4 \times 10^9$ |

Aus den vorstehenden Daten ist zu ersehen, daß sich bei Anwesenheit der cholesterinreichen bovinen Fraktion die Gesamt-Zahl der Zellen verdoppelte, die Zahl der lebensfähigen hundertfach erhöhte und eine positive Wirkung auf die Zellmorphologie feststellen ließ.

Beispiel 2

Impfkulturen von T. hyodysenteriae ATCC Nr. 31212, die wie im vorstehenden Beispiel 1 beschrieben hergestellt wurden, wurden dann zur Beimpfung der Inhalte von drei 500 ml-Flaschen mit einer Konzentration von 3 Vol.-% verwendet, die jeweils 200 ml tryptischer Sojabrühe vermischt mit 1 % Hefeextrakt, 3 % Lammserum, 1 % Dextrose, 0,05 % L-Cystein-HCl und 0,25 µg/ml Vitamin B 12 enthielten. Die Prozentzahlen beziehen sich auf Gewicht/Volumen. Eine der Flaschen (Kontrolle) enthielt 2,5 Vol.-% der wie in Beispiel 1 beschrieben hergestellten cholesterinreichen bovinen Fraktion. Eine zweite Flasche enthielt 2,5 Vol.-% Cholesterol Concentrate Code 82-010 von Miles Laboratories, Inc.. Eine dritte Flasche enthielt 2,5 Vol.-% Bovine Cholesterol Concentrate List 3200 von Biocell Laboratories. Diese beimpften Flaschen wurden dann unter einer anaeroben Atmosphäre aus 10 Vol.-% Wasserstoff, 80 Vol.-% Stickstoff und 10 Vol.-% Kohlenstoffdioxid 38 h bei 37°C inkubiert. Die Inhalte der Flaschen wurden bei 24 h und bei 31 h durch Zusatz von 2N Natriumhydroxid auf pH 7,0 eingestellt. Die Gesamt-Zahl der Zellen aus jeder Flasche wurde in einer Petroff-Hauser-Zählkammer bestimmt.Die Ergebnisse sind in der folgenden Tabelle 2 zusammengestellt.

## Tabelle 2

<u>Wachstum von T. hyodysenteriae ATCC 31212 in Medien mit</u>
<u>verschiedenartigen cholesterinreichen bovinen Fraktio-</u>
<u>nen</u>

| Ergänzung des Mediums | Gesamt-Zahl der Zellen |
|---|---|
| Bevorzugte Cholesterin-Fraktion (Kontrolle) | $2,5 \times 10^9$/ml |
| Cholesterol Concentrate Code 82-010 | $2,7 \times 10^9$/ml |
| Bovine Cholesterol Concentrate List 3200 | $3,0 \times 10^9$/ml |

Aus den vorstehenden Daten ist zu ersehen, daß cholesterinreiche bovine Fraktionen aus verschiedenen Quellen zur Erzeugung eine wünschenswert hohen Zell- Zahl von T. hyodysenteriae eingesetzt werden können.

Beispiel 3

Die Arbeitsweise des Beispiels 1 wurde allgemein wiederholt, wobei T. hyodysenteriae Stamm ATCC Nr. 31287 mit dem durch die bevorzugte Cholesterin-Fraktion ergänzten Medium zur Erzeugung einer hohen Konzentration an Zellen eingesetzt wurde.

Beispiel 4

Ein Impfstoff wurde hergestellt durch Vermischen der abgetöteten T. hyodysenteriae-Zellen, die in der in Beispiel 1 beschriebenen Weise unter Einsatz der bevorzugten Cholesterin-Fraktion in dem Wachstumsmedium gewonnen worden waren, mit 10 Vol.-% des in der US-PS 3 919 411 beschriebenen Adjuvans HAVLOGEN® und 1 : 10 000 Merthiolat. Der Impfstoff enthielt $2 \times 10^9$ Zellen/ml. Dieser Impfstoff wurde dann in fünf getrennten Immunitätstests zur Bestimmung der Wirksamkeit des Impfstoffs gegen Schweine-Dysenterie verwendet. In jedem dieser Tests der Impfung/Immunität wurden die Schweine aus einer Herde genommen, in der bisher keine Fälle von Schweine-Dysenterie aufgetreten war; es handelte sich um Schweine beiderlei Geschlechts und im allgemeinen um solche der Yorkshire- und Hampshire-Rassen oder aus Kreuzungszuchten. Zum Zeitpunkt der ersten Impfung waren die Schweine wenigstens bereits drei Wochen abgesetzt und hatten ein Gewicht im Bereich von 16 bis 18 kg (35 bis 40 lbs.).

Sämtliche Tests der Impfung/Immunität wurden in einer Isolierstation durchgeführt, die eine Abtrennung der geimpften Schweine und der Kontrolltiere ermöglichte. Die Schweine erhielten die Proteinzunahme bewirkendes Futter ohne Antibiotika. Vor dem Immunitätstest wurde durch rektale Abstriche und Tests auf geeigneten Isoliermedien sichergestellt, daß die Schweine frei von T. hyodysenteriae und von Salmonella spp. waren.

Die Schweine wurden zum Immunitätstest intragastral mit den T. hyodysenteriae-Stämmen ATCC Nr. 31212 oder 31287 infiziert, die mittels einer Arbeitsweise ähnlich derjenigen von Beispiel 1 vermehrt worden waren, jedoch mit der Abweichung, daß die Zellen zum Schluß nicht abgetötet wurden. Die Infektionsdosis bestand aus 100 ml aktiver Kultur, die so verdünnt worden war, daß das Volumen $10^8$ bis $10^9$ Organismen enthielt; sie wurde den einzelnen Schweinen mittels Magensonde nach 48-stündigem Nahrungsentzug verabreicht.

Jedes dem Immunitätstest unterzogene Schwein wurde während eines Zeitraums von 28 Tagen nach der Infizierung täglich beobachtet. Nach dem Ausbruch der klinischen Erkrankung wurden Schweinen mit Dysenterie rektale Abstriche zur Isolierung des verurachenden Agens entnommen. Die Bestätigung der T. hyodysenteriae-Infektion erfolgte durch Subkultivieren der Abstriche auf Spectinomycin-Blutagar-Platten, 4- bis 6-tägiges Inkubieren bei 42°C und mikroskopische Untersuchung hämolytischer Agar-Plaques auf die Anwesenheit von Spirochäten. An jedem Schwein, das während der fünf Untersuchungen verstarb, wurde eine Autopsie durchgeführt. Makroskopische Läsionen wurden verzeichnet, und rektale Abstriche wurden der Colon-Schleimhaut entnommen und wie oben angegeben subkultiviert. Die klinische Reaktion jedes einzelnen Schweins auf die Immuntest-Infektion wurde mit Hilfe des nachstehenden klinischen Index gemessen:

<u>Klinischer Index</u>

| Allgemeiner Zustand | Fäces: Zusammensetzung | Fäces: Konsistenz |
|---|---|---|
| 0 – Normal | 0 – Normal | 0 – Normal |
| 1 – Diarrhöe | 1 – Schleim | 1 – Weich |
| 2 – Dysenterie | 2 – Blut und Schleim | 1,5 – Locker |
| 3 – Abgemagert | | 2 – Flüssig |
| 4 – Moribund | 3 – Blut | 3 – Wäßrig |
| 5 – Tot | | |

Verschiedene Methoden der Analyse wurden zur Auswertung klinischer Index-Werte angewandt; sie werden nachstehend definiert:

a) Täglicher klinischer Index (DCI):
Ein täglicher klinischer Index wurde für jedes im Immuntest befindliche Schwein mit Hilfe der drei oben beschriebenen Parameter berechnet.

DCI = Allgemeiner Zustand + Zusammensetzung der Fäces + Konsistenz der Fäces

b) Täglicher klinischer Gruppen-Index (DGCI):
Der tägliche klinische Gruppen-Index für geimpfte Gruppen und Kontroll-Gruppen wurde mittels der folgenden Formel berechnet:

$$DGCI = \frac{\sum SDCI \text{ (für alle Schweine einer Gruppe)}}{\text{Zahl der Schweine in einer Gruppe}} \times 100$$

c) Individueller kumulativer klinischer Index (ICCI):
Ein kumulativer klinischer Index wurde für jedes Schwein über den gesamten Zeitraum von 28 Tagen nach dem Beginn des Immunitätstests wie folgt berechnet:

$$ICCI = \frac{\sum SDCI \text{ (für die 28 Tage nach Infektion)}}{\text{Gesamtzahl der Beobachtungen (84)}} \times 100$$

d) Kumulativer klinischer Gruppen-Index (GCCI):
Ein kumulativer klinischer Gruppen-Index für geimpfte Gruppe und Kontroll-Gruppe wurde durch Mittelwertbildung der ICCI-Werte innerhalb einer speziellen Gruppe von Schweinen erhalten:

7

$$\text{GCCI} = \frac{\sum \text{SICCI(für alle Schweine einer Gruppe)}}{\text{Zahl der Schweine in einer Gruppe}} \times 100$$

Untersuchung Nr. 1:

10 Schweine wurden gleichmäßig auf zwei Ställe verteilt. Fünf Tiere der Impfgruppe erhielten zwei intramuskuläre Injektionen von je 5 ml des oben beschriebenen Impfstoffes im Abstand von drei Wochen. Fünf ungeimpfte Tiere der Kontrollgruppe wurden in einem abgetrennten Stall gehalten. Zwei Wochen nach der Wiederholungsimpfung wurden sämtliche Schweine dem Immunitätstest durch Infektion mit virulentem T. hyodysenteriae Stamm ATCC 31212 unterzogen.

Untersuchung Nr. 2:

29 Schweine wurden auf fünf Ställe mit 23 Tieren der Impfgruppe und zwei Ställe mit 6 Kontrolltieren verteilt. Die Tiere der Impfgruppe erhielten zwei intramuskuläre Injektionen von je 5 ml des oben beschriebenen Impfstoffes im Abstand von drei Wochen. Die Immunitätsprüfung war die gleiche wie in der Untersuchung Nr. 1.

Untersuchung Nr. 3:

10 Schweine wurden gleichmäßig auf zwei Ställe verteilt. Fünf Tiere der Impfgruppe erhielten zwei intramuskuläre Injektionen von je 5 ml des oben beschriebenen Impfstoffes im Abstand von drei Wochen. Fünf Tiere wurden als ungeimpfte Kontrolltiere gehalten. Zwei Wochen nach der Wiederholungsimpfung wurden sämtliche Schweine dem Immunitätstest durch Infektion mit virulentem T. hyodysenteriae Stamm ATCC 31287 unterzogen.

Untersuchung Nr. 4:

46 Schweine wurden auf neun Ställe mit 36 Tieren der Impfgruppe und zwei Ställe mit 10 ungeimpften Kontrolltieren verteilt. Die Schweine der Impfgruppe erhielten zwei intramuskuläre Injektionen von je 5 ml des oben beschriebenen Impfstoffes im Abstand von drei Wochen. Die Immunitätsprüfung war die gleiche wie in der Untersuchung Nr. 3.

Untersuchung Nr. 5:

46 Schweine wurden auf neun Ställe mit 36 Tieren der Impfgruppe und zwei Ställe mit 10 ungeimpften Kontrolltieren verteilt. Die Schweine der Impfgruppe erhielten zwei intramuskuläre Injektionen von je 5 ml des oben beschriebenen Impfstoffes im Abstand von drei Wochen. Die Immunitätsprüfung war die gleiche wie in der Untersuchung Nr. 1.

Die fünf Untersuchungen erstreckten sich auf eine Gesamtzahl von 105 Impftieren und 36 Kontrolltieren. Die Ergebnisse der fünf Untersuchungen sind in den Tabellen 3 und 4 zusammengefaßt.

## Tabelle 3

**Klinische Reaktion von Schweinen im Immunitätstest aufgrund gastraler Infektion mit T. hyodysenteriae ATCC 31212**

| | Geimpfte Tiere | | | | Kontrolltiere | | | |
| | Untersuchung Nr. | | | Total | Untersuchung Nr. | | | Total |
| | 1 | 2 | 5 | | 1 | 2 | 5 | |
|---|---|---|---|---|---|---|---|---|
| Zahl der Schweine | 5 | 23 | 36 | 64 | 5 | 6 | 10 | 21 |
| **Klinische Dysenterie** | | | | | | | | |
| Zahl der Fälle | 1 | 8 | 2 | 11 | 5 | 4 | 6 | 15 |
| Prozent | 20 | 34,7 | 5,5 | 17,2 | 100 | 66,7 | 60 | 71,4 |
| Mittlerer Tag des Ausbruchs | 10 | 12,6 | 15,5 | 12,9 | 10,8 | 7,0 | 10,3 | 9,6 |
| Mittlere Dauer in Tagen | 5 | 3,5 | 3,5 | 3,6 | 12,9 | 8,4 | 6,6 | 10,3 |
| **Abmagerung** | | | | | | | | |
| Zahl der Fälle | 0 | 1 | 0 | 1 | 0 | 0 | 1 | 1 |
| Prozent | 0 | 4,3 | 0 | 1,6 | 0 | 0 | 10 | 4,8 |
| **Tod** | | | | | | | | |
| Zahl der Fälle | 0 | 0 | 0 | 0 | 3 | 1 | 1 | 5 |
| Prozent | 0 | 0 | 0 | 0 | 60 | 16,7 | 10 | 23,8 |
| **Anorexie** | | | | | | | | |
| Tage | 0 | 1 | 0 | 1 | 9 | 4 | 2 | 15 |
| **Klinischer Gruppen-Index** | 8,8 | 23,3 | 2,3 | 10,4 | 159,0 | 82,3 | 45,9 | 83,2 |

Nach der intragastralen Infektion mit Stamm ATCC Nr. 31212 im Immunitätstest wurde klinische Dysenterie bei 17,2 % der geimpften Schweine (11 von 64) und bei 71,4 % der Kontrollschweine (15 von 21) beobachtet. Dies bezeichnet eine Reduktion von 75,9 % der tatsächlichen Fälle klinischer Dysenterie unter den geimpften Schweinen. Der für die gesamte Beobachtungsperiode nach der Test-Infektion von 28 Tagen berechnete kumulative klinische Index (GCCI) betrug für die geimpften Schweine 10,4 im Vergleich zu 83,2 für die ungeimpften Kontrollen. Dies bezeichnet eine Reduktion von 87,5 % der klinischen Anzeichen insgesamt (Diarrhöe, Dysenterie, Abmagerung, Tod), wie dies durch den klinischen Index gemessen wurde. Keine Todesfälle wurden unter den geimpften Schweinen verzeichnet, während 5 von 21 Kontrollschweinen (23,8 %) nach der Test-Infektion starben. Der Ausbruch der klinischen Dysenterie war bei den geimpften Schweinen verzögert, und die Dauer der klinischen Erkrankung war verkürzt. Die Anorexie war bei den geimpften Schweinen beträchtlich reduziert.

## Tabelle 4

### Klinische Reaktion von Schweinen im Immunitätstest aufgrund gastraler Infektion mit T. hyodysenteriae ATCC 31287

|  | Geimpfte Tiere | | | Kontrolltiere | | |
|  | Untersuchung Nr. | | Total | Untersuchung Nr. | | Total |
|  | 3 | 4 | | 3 | 4 | |
|---|---|---|---|---|---|---|
| Zahl der Schweine | 5 | 36 | 41 | 5 | 10 | 15 |
| Klinische Dysenterie | | | | | | |
| Zahl der Fälle | 2 | 12,5 | 14,5 | 3 | 7,5 | 10,5 |
| Prozent | 40 | 34,7 | 35,4 | 60 | 75 | 70 |
| Mittlerer Tag des Ausbruchs | 11,5 | 13,6 | 13,3 | 6,3 | 6,6 | 6,6 |
| Mittlere Dauer in Tagen | 3,0 | 2,5 | 2,6 | 15,3 | 6,4 | 8,9 |
| Abmagerung | | | | | | |
| Zahl der Fälle | 0 | 0 | 0 | 1 | 1 | 2 |
| Prozent | 0 | 0 | 0 | 20 | 10 | 13,3 |
| Tod | | | | | | |
| Zahl der Fälle | 0 | 0 | 0 | 1 | 1 | 2 |
| Prozent | 0 | 0 | 0 | 20 | 10 | 13,3 |
| Anorexie | | | | | | |
| Tage | 0 | 0 | 0 | 4 | 0 | 4 |
| Klinischer Gruppen-Index | 21,2 | 18,2 | 18,6 | 93,2 | 63,6 | 73,5 |

Nach der intragastralen Infektion mit Stamm ATCC Nr. 31287 im Immunitätstest wurde klinische Dysenterie bei 35,4 % der geimpften Schweine (14 von 41, 1 Schwein ±) und bei 70,0 % der Kontrollschweine (10 von 15, 1 Schwein ±) beobachtet. Dies bezeichnet eine Reduktion von 49,4 % der tatsächlichen Fälle klinischer Dysenterie unte den geimpften Schweinen. Der kumulative klinische Index betrug für die geimpften Schweine 18,6 im Vergleich zu 73,5 für die ungeimpften Kontrollen. Dies bezeichnet eine Reduktion von 74,7 % der klinischen Anzeichen insgesamt bei den Geimpften. Keine Todesfälle wurden unter den geimpften Schweinen verzeichnet, während 2 von 15 Kontrollschweinen (13,3

10

%) nach der Test-Infektion starben. Bei den geimpften Schweinen waren der Ausbruch der klinischen Dysenterie signifikant verzögert und die Dauer der klinischen Erkrankung verkürzt.

Die Bestätigung dafür, daß T. hyodysenteriae das ursächliche Agens der klinischen Dysenterie war, wurde in sämtlichen Fällen nach dem Ausstreichen der rektalen Abstriche auf Spectinomycin-Blutagar-Platten erhalten. Das gleichzeitige Ausstreichen auf für Salmonellen spp. selektive Medien lieferte negative Ergebnisse.

Die Dickdarm-Inhalte der toten Schweine waren blutig und wäßrig, und die Dickdärme selbst waren weitgehend entzündet und frei von Villi. Von den Darmwänden bei der Autopsie entnommene Abstriche waren positiv für T. hyodysenteriae und negativ für Salmonella spp.. Rektal-Abstriche die 28 Tage nach der Test-Infektion von geschützten Impflingen entnommen wurden, waren nach der Subkultur auf Spectinomycin-Blutagar-Platten bei 42°C in allen Fällen negativ.

Berücksichtigt man alle 141 geimpften Schweine und Kontrolltiere, so läßt sich die Tabelle 5 erstellen, die die klinischen Ergebnisse sämtlicher fünf Immunitätstests zusammenfaßt. 25,5 von 105 geimpften Tieren entwickelten klinische Dysenterie (24,3 %), während 25,5 von 36 ungeimpften Kontrolltieren klinische Dysenterie entwickelten (70,8 %). Dies bezeichnet eine Reduktion von 65,7 % der Fälle klinischer Dysenterie unten allen geimpften Schweinen. 7 von 36 Kontroll-Schweinen starben nach der Test-Infektion (19,4 %), während von den geimpften Schweinen nach der Test-Infektion keine Tiere starben. Der kumulative klinische Gruppen-Index betrug für alle 105 geimpften Schweine 13,6; der GCCI für 36 ungeimpfte Kontrollen beträgt 79,2. Dies läßt sich ausdrücken als eine Reduktion von 82,8 % der klinischen Anzeichen insgesamt (Diarrhöe, Dysenterie, Abmagerung, Tod) bei der Gesamtheit der geimpften Schweine.

Berücksichtigt man die Ergebnisse sämtlicher Impf-Immunitätstests aus den fünf einzelnen Untersuchungen, so ist daraus schließen, daß der T. hyodysenteriae-Impfstoff einen definitiven Wirkungsgrad als Mittel zur Verhütung der Schweine-Dysenterie beim Einsatz unter Feldbedingungen zeigt.

## Tabelle 5

<u>Immunitätstest-Untersuchungen Nr. 1, 2, 3, 4, 5 -</u>
<u>Klinische Reaktion von Schweinen im Immunitätstest</u>
<u>aufgrund gastraler Infektion mit T. hyodysenteriae</u>
<u>ATCC 31212 und 31287</u>

|  | <u>Geimpfte</u> <u>Tiere</u> | <u>Kontroll-</u> <u>tiere</u> |
|---|---|---|
| Zahl der Schweine | 105 | 36 |
| <u>Klinische</u> <u>Dysenterie</u> Zahl der Fälle | 25,5 | 25,5 |
| Prozent | 24,3 | 70,8 |
| Mittlerer Tag des Ausbruchs | 13,1 | 8,2 |
| Mittlere Dauer in Tagen | 2,8 | 9,1 |
| <u>Abmagerung</u> Zahl der Fälle | 1 | 3 |
| Prozent | 1,0 | 8,3 |
| <u>Tod</u> Zahl der Fälle | 0 | 7 |
| Prozent | 0 | 19,4 |
| <u>Anorexie</u> Tage | 1 | 21 |
| <u>Klinischer</u> <u>Gruppen-Index</u> | 13,6 | 79,2 |

**Patentansprüche**

1. Verfahren zur Herstellung eines Impfstoffs auf Basis von Treponema hyodysenteriae, dadurch gekennzeichnet, daß Treponema hyodysenteriae in einem Medium vermehrt wird, das eine cholesterinreiche bovine Fraktion enthält, die mittels eines Verfahrens hergestellt ist, das die folgenden Schritte umfaßt:

   (a) In-Berührung-Bringen eines flüssigen, Cholesterin enthaltenden bovinen Plasmas oder Serums oder einer Fraktion daraus mit einem Siliciumdioxid-Adsorptionsmittel, um das Cholesterin zu adsorbieren;

   (b) Abtrennen des adsorbierten Cholesterins aus dem verbleibenden flüssigen Plasma oder Serum;

(c) Einfrieren und Auftauen des adsorbierten Cholesterins;

(d) Eluieren des adsorbierten Cholesterins bei einem pH von 9,0 bis 11,5;

(e) Konzentrieren der eluierten Cholesterin-Lösung durch Ultrafiltration;

(f) Einstellen des pH der konzentrierten Cholesterin-Lösung auf einen Wert im Bereich von 11,0 bis 11,4;

(g) Dialysieren der konzentrierten Cholesterin-Lösung nacheinander gegen Natriumcarbonat und Wasser;

(h) weiteres Konzentrieren der dialysierten Cholesterin-Lösung durch Ultrafiltration auf einen Cholesterinspiegel von 50 bis 3000 mg/dl;

(i) Einstellen des pH der konzentrierten Cholesterin-Lösung auf einen Wert im Bereich von 7,0 bis 11,0;

(j) Erhitzen der konzentrierten Cholesterin-Lösung auf 50°C bis 100°C für die Dauer von 30 min bis 24 h; und

(k) Isolieren einer gereinigten cholesterinreichen bovinen Fraktion daraus.

2. Verfahren nach Anspruch 1, dadurch gekennzeichnet, daß das Nährmedium 0,1 bis 5,0 Vol.-% der cholesterinreichen bovinen Fraktion enthält.

3. Verfahren nach Anspruch 1, dadurch gekennzeichnet, daß das Nährmedium 1 bis 2,5 Vol.-% der cholesterinreichen bovinen Fraktion enthält.

4. Verfahren nach Anspruch 1, dadurch gekennzeichnet, daß das Nährmedium 2,5 Vol.-% der cholesterinreichen bovinen Fraktion enthält.

5. Verfahren nach Anspruch 1, dadurch gekennzeichnet, daß das Nährmedium 1 bis 2,5 Vol.-% einer cholesterinreichen bovinen Fraktion enthält, die mittels eines Verfahrens hergestellt ist, das die folgenden Schritte umfaßt:

(a) In-Berührung-Bringen eines flüssigen, Cholesterin enthaltenden bovinen Plasmas oder Serums oder einer Fraktion daraus mit einem Siliciumdioxid-Adsorptionsmittel, um das Cholesterin zu adsorbieren;

(b) Abtrennen des adsorbierten Cholesterins aus demverbleibenden flüssigen Plasma oder Serum;

(c) Einfrieren und Auftauen des adsorbierten Cholesterins;

(d) Eluieren des adsorbierten Cholesterins bei einem pH von 10,4 bis 10,6;

(e) Konzentrieren der eluierten Cholesterin-Lösung durch Ultrafiltration auf ein Volumen von 15 bis 50 % des Ausgangsvolumens;

(f) Einstellen des pH der konzentrierten Cholesterin-Lösung auf einen Wert von 11,2;

(g) Dialysieren der konzentrierten Cholesterin-Lösung nacheinander gegen Natriumcarbonat und Wasser;

(h) weiteres Konzentrieren der dialysierten Cholesterin-Lösung durch Ultrafiltration auf einen Cholesterinspiegel von 1000 bis 2000 mg/dl;

(i) Einstellen des pH der konzentrierten Cholesterin-Lösung auf einen Wert von 7,6;

(j) Erhitzen der konzentrierten Cholesterin-Lösung auf 80°C für die Dauer von 30 bis 60 min; und

(k) Isolieren einer gereinigten cholesterinreichen bovinen Fraktion daraus.

## Claims

1. Method for the preparation of a vaccine based on Treponema hyodysenteriae, characterised in that Treponema hyodysenteriae is replicated in a medium which contains a cholesterol-rich bovine fraction which is prepared by means of a method which comprises the following steps:

(a) bringing a liquid, cholesterol-containing bovine plasma or serum or a fraction thereof into contact with a silica adsorbent in order to adsorb the cholesterol;

(b) removal of the adsorbed cholesterol from the remaining liquid plasma or serum;

(c) freezing and thawing the adsorbed cholesterol;

(d) elution of the adsorbed cholesterol at a pH of 9.0 to 11.5;

(e) concentration of the eluted cholesterol solution by ultrafiltration;

(f) adjustment of the pH of the concentrated cholesterol solution to a value in the range from 11.0 to 11.4;

(g) dialysis of the concentrated cholesterol solution successively against sodium carbonate and water;

(h) further concentration of the dialysed cholesterol solution by ultrafiltration to a cholesterol level of 50 to 3000 mg/dl;

(i) adjustment of the pH of the concentrated cholesterol solution to a value in the range from 7.0 to 11.0;

(j) heating of the concentrated cholesterol solution at 50°C to 100°C for a period of 30 min to 24 h; and

(k) isolation of a purified cholesterol-rich bovine fraction therefrom.

2. Method according to Claim 1, characterised in that the nutrient medium contains 0.1 to 5.0% by volume of the cholesterol-rich bovine fraction.

3. Method according to Claim 1, characterised in that the nutrient medium contains 1 to 2.5% by volume of the cholesterol-rich bovine fraction.

4. Method according to Claim 1, characterised in that the nutrient medium contains 2.5% by volume of the cholesterol-rich bovine fraction.

5. Method according to Claim 1, characterised in that the nutrient medium contains 1 to 2.5% by volume of a cholesterol-rich bovine fraction which is prepared by means of a method which comprises the following steps:

(a) bringing a liquid, cholesterol-containing bovine plasma or serum or a fraction thereof into contact with a silica adsorbent in order to adsorb the cholesterol;

(b) removal of the adsorbed cholesterol from the remaining liquid plasma or serum;

(c) freezing and thawing the adsorbed cholesterol;

(d) elution of the adsorbed cholesterol at a pH of 10.4 to 10.6;

(e) concentration of the eluted cholesterol solution by ultrafiltration to a volume of 15 to 50% of the starting volume;

(f) adjustment of the pH of the concentrated cholesterol solution to a value of 11.2;

(g) dialysis of the concentrated cholesterol solution successively against sodium carbonate and water;

(h) further concentration of the dialysed cholesterol solution by ultrafiltration to a cholesterol level of 1000 to 2000 mg/dl;

(i) adjustment of the pH of the concentrated cholesterol solution to a value of 7.6:

(j) heating of the concentrated cholesterol solution at 80°C for a period of 30 to 60 min; and

(k) isolation of a purified cholesterol-rich bovine fraction therefrom.

**Revendications**

1. Procédé de préparation d'un vaccin à base de Treponema hyodysenteriae caractérisé en ce que le Treponema hyodysenteriae est multiplié dans un milieu qui contient une fraction bovine riche en cholestérol préparée par un procédé qui comprend les étapes suivantes:

(a) mise en contact d'un plasma ou d'un sérum bovin liquide ou de leur fraction contenant du cholestérol avec un agent d'adsorption de dioxyde de silicium pour adsorber le cholestérol;

(b) la séparation du cholestérol adsorbé du plasma ou sérum liquide résiduel;

(c) la congélation et le réchauffement du cholestérol adsorbé;

(d) l'élution du cholestérol adsorbé à un pH de 9,0 à 11,5;

(e) la concentration de la solution de cholestérol éluée par ultrafiltration;

(f) le réglage d'un pH de la solution de cholestérol concentrée à une valeur de l'ordre de 11,0 à 11,4;

(g) la dialyse de la solution de cholestérol concentrée contre, successivement, du carbonate de sodium et de l'eau;

(h) une nouvelle concentration de la solution de cholestérol dialysée par ultrafiltration à un taux de cholestérol de 50 à 3000 mg/dl;

(i) le réglage du pH de la solution de cholestérol concentrée à une valeur de l'ordre de 7,0 à 11,0;

(j) le chauffage de la solution de cholestérol concentrée à une température de 50 à 100°C pendant 30 min à 24 h;

(k) l'isolation d'une fraction bovine purifiée riche en cholestérol.

**2.** Procédé selon la revendication 1 caractérisé en ce que le milieu nutritif contient 0,1 à 5,0 % en vol. de fraction bovine riche en cholestérol.

**3.** Procédé selon la revendication 1 caractérisé en ce que le milieu nutritif contient 1 à 2,5 % en vol. de fraction bovine riche en cholestérol.

**4.** Procédé selon la revendication 1 caractérisé en ce que le milieu nutritif contient 2,5 % en vol. de la fraction bovine riche en cholestérol.

**5.** Procédé selon la revendication 1 caractérisé en ce que le milieu nutritif contient 1 à 2,5 % en vol. d'une fraction bovine riche en cholestérol qui est préparée à l'aide d'un procédé qui comprend les étapes suivantes:

(a) mise en contact d'un plasma ou sérum bovin liquide ou de leur fraction contenant du cholestérol avec un agent d'adsorption de dioxyde de silicium pour adsorber le cholestérol;

(b) la séparation du cholestérol adsorbé du plasma ou sérum liquide résiduel;

(c) la congélation et le réchauffement du cholestérol adsorbé;

(d) l'élution du cholestérol adsorbé à un pH de 10,4 à 10,6;

(e) la concentration de la solution de cholestérol éluée par ultrafiltration à un volume de 15 à 50 % du volume initial;

(f) le réglage d'un pH de la solution de cholestérol concentrée à une valeur de 11,2;

(g) la dialyse de la solution de cholestérol concentrée contre successivement du carbonate de sodium et de l'eau;

(h) une nouvelle concentration de la solution de cholestérol dialysée par ultrafiltration à un taux de cholestérol de 1000 à 2000 mg/dl;

(i) le réglage du pH de la solution de cholestérol concentrée à une valeur de l'ordre de 7,6;

(j) le chauffage de la solution de cholestérol concentrée à une température de 80 °C pendant 30 à 60 min; et

(k) l'isolation d'une fraction bovine purifiée riche en cholestérol.